# EUROPEAN PATENT APPLICATION

(11) **EP 2 901 957 A1**
(43) Date of publication of application: **05.08.2015**
(21) Application number: 14153412.3
(22) Date of filing: 31.01.2014
(51) Int. Cl.: A61B 19/00

(54) **Controlling a surgical intervention to a bone**

(71) Applicant: Universität Basel, 4003 Basel (CH); Universitätsspital Basel, 4031 Basel (CH)
(72) Inventor: Cattin, Philippe, 5210 Windisch (CH); Jost, Gregory, 4102 Binningen (CH); Walti, Jonas, 3626 Hünibach (CH)
(74) Representative: Latscha Schöllhorn Partner AG

(57) **Abstract**

A method of controlling a surgical intervention to a bone (31) comprises: obtaining a three-dimensional image or multiplanar reconstruction of the bone (31), defining a position and an axis of intervention on the three-dimensional image or multiplanar reconstruction of the bone (31), and controlling the orientation of an intervention instrument (1) equipped with an orientation sensor (2) during the surgical intervention by evaluating a signal provided by the orientation sensor (2). The method further comprises referencing the intervention instrument (1) with respect to the bone (31) before the surgical intervention by arranging the intervention instrument (1) along an anatomic landmark (32) being an edge of the bone (31) and rotating the orientation sensor (2) into a predefined position, or by arranging the intervention instrument (1) essentially perpendicular to an anatomic landmark (32) being an essentially flat surface of the bone (31) and rotating the orientation sensor (2) into a predefined position. The method according to the invention allows for efficiently achieving correct orientation and position of the intervention instrument in a bone surgical intervention. Furthermore, the method according to the invention provides an efficient real-time control at comparably low efforts. Still further, with the method according to the invention radiation exposure to the operational staff as well as patients can be reduced.

## Description

### Technical Field

The present invention relates to a method of controlling a surgical intervention to a bone according to the preamble of independent claim 1 and more particularly to a surgical intervention system and a method of a surgical intervention to a bone using such a surgical intervention system.

Such methods of controlling a surgical intervention to a bone, in which a three-dimensional image of the bone is obtained, a position and an axis of intervention on the three-dimensional image of the bone is defined and the orientation of an intervention instrument fixedly equipped with an orientation sensor during the surgical intervention by evaluating a signal provided by the orientation sensor is controlled, can be used for providing a comparably precise, predictable and safe intervention to the bone.

### Background Art

In many surgical interventions applied to bones, intervention instruments or surgical tools such as drills, awls, screwdrivers or the like are involved. For applying such instruments or tools their position and orientation often is crucial for the success of the surgical intervention.

For example, in dental surgery it can be important to assure that dental implants are arranged in a predefined orientation to each other. For that purpose, it is known to track the orientation of tools preparing the implantation. WO 2011/089606 A1 describes a hand-held dental tool which comprises an orientation detector. Before the tool is applied it is manually positioned in a predefined orientation and this orientation is stored by pressing a reference button. Like this, a dentist can for example arrange the tool in line with an existing first bore and store the orientation in order to apply a second bore parallel to the first one. When applying the tool the orientation detector detects the current orientation which is compared to the predefined orientation and any deviation is shown on a display. Whereas such a tool can be helpful for dental or similar applications it is not suitable for other applications. In particular, the manual referencing of the tool is in many applications not possible since no corresponding reference application exists. Furthermore, in some surgical interventions it is not sufficient to control orientation of the tool but its position has also to be involved.

As another example of surgical intervention, in spine surgery often screws are placed in vertebras in order to stabilize or support the spine or in order to fix auxiliary structures for particular purposes. Thereby, warranting the correct trajectory to implant a screw without harming neurovascular structures depends on obtaining a pathway with the correct starting point and tilt in the sagittal and axial plane. Different tactics can lead to successful placement of a screw in the spine. A technique widely used to instrument the spine via an open access involves the following steps, wherein surgeons may modify some of the steps to suit their preferences. (i) The spine is exposed at the levels of interest and anatomical landmarks are identified on the spine. (ii) The landmarks guide the surgeon to the entry points (for instance for implanting pedicle screws), and the bone at the entry point is decorticated, for instance by drilling a little pilot hole. (iii) In the case of implanting a pedicle screw, a pedicle finder is used to cannulate the pedicle through the pilot hole. A lateral fluoroscopy at the beginning of this process ensures that the entry point is chosen at the correct point in the cephalo-caudal direction, i.e. not too high up towards the head and not too inferior towards the feet, and that the sagittal tilt is ok. If necessary, entry point, sagittal orientation or both are immediately corrected. (iv) The pedicle finder is advanced into the pedicle and the tactile feedback helps judging its progress into the cancellous bone of the vertebral body. One or two lateral fluoroscopies are made to track advancement of the pedicle finder. Fluoroscopy corresponds to image guidance or navigation in the sagittal plane and per se informs the surgeon in real-time of the sagittal tilt. (v) During advancement, the surgeon must also respect tilt or angulation in the axial plane. Aiming too medial will breach the medial pedicle wall and potentially harm nerve roots in the lumbar spine or the spinal cord in the thoracic spine. Aiming too laterally will position the screw lateral to the vertebral body and hence lack any bone purchase. Moreover, laterally placed screws in the thoracic spine may jeopardize the aorta or vena cava.

In open spine surgeries, the starting point is usually defined by exposed anatomic landmarks, but the level within the spine and sagittal tilt of the vertebral body with respect to the surgical instruments and their advancement into the bone are usually controlled with intraoperative mostly lateral fluoroscopy. After observation of the preoperative imaging, the axial tilt is commonly chosen by feel and experience. Hence the procedure is associated with considerable subjective control.

Also, in many cases the vertebral anatomy is not exposed entirely but most of the crucial vertebral architecture is inferred from exposed landmarks such as transverse processes, facet joints, isthmus, lamina, spinous process, etc.. Particularly in such cases, some of the hidden anatomy is visualized with fluoroscopy. Although the classic use of a C-arm fluoroscope in spine surgery is a form of image-guided surgery, this is usually limited to a lateral monoplanar image. Biplanar imaging can add visual information in the coronal plane but its use is cumbersome as two C-arms need to be setup which impairs the surgeon's ability to freely move about the surgical field, or alternatively, one C-arm needs to be flipped back and forth which raises concerns of sterility.

In this context, the term "intraoperative navigation" or "image-guidance" describes any additional or comparably sophisticated equipment that resolves the limits of, e.g., static images restricted to one plane at a time within common fluoroscopy and hence provides the surgeon with more information about anatomy and position of instruments and tools. Modern image guidance delivers real-time visual feedback in three planes and minimizes radiation exposure to the operational staff by eliminating the need for repeated radiation after the initial image acquisition. The center of a contemporary image-guidance system usually is a workstation computer that computes real-time images of the anatomy surrounding the tip of the navigated instrument or tool. This instrument tool bears visual (reflectors, light-emitting diodes), acoustic (ultrasoundemitting transducers), or electromagnetic sensors which are tracked by a stereoscopic camera, microphone, or electromagnetic transmitter. Real-time images usually are reconstructed from the stored image data set by comparing the spatial relationship of the surgeon's instruments with a rigidly attached reference array on the patient's spine.

Thus, contemporary image guidance often tracks the position of surgical tools in relation to the anatomy and renders a real-time visualization in three planes on a screen of a workstation. These devices can be suitable tools which may improve accuracy of spine surgery or spinal instrumentation and reduce radiation exposure to the operational staff and possibly the patient. However, their high cost precludes availability to most surgeons. Also these devices usually involve a comparably complex setup procedure including the following: (i) All instruments that have to be tracked during the surgery require calibration with the workstation. (ii) A radiographic or similar image comprising the anatomy of interest has to be transferred to the workstation. This can be a preoperative computer tomography scan or an intraoperative image such as 2D- or 3D-fluoroscopy. (iii) A dynamic reference array is fixed to the spine, and predefined landmarks on the patient's actual anatomy are touched with a tracked probe (paired points registration) as well as a different set of random points on the spine surface (surface matching registration). Some systems using intraoperative image acquisition have an incorporated reference array that is tracked during image acquisition. Since the workstation then calculates the spatial relationships between the imaging machine, the dynamic reference array on the spine, and the acquired image data set, a manual registration process can be omitted. A third option for registration is the 2D-3D merge in which the workstation merges the anteroposterior and lateral fluoroscopic images of the positioned patient with the preoperative computer tomography. This is an automated registration process without the need for surface matching and therefore like 3D-fluoroscopy or intraoperative computer tomography scanning applicable to percutaneous procedures. (iv) Anatomic landmarks are again touched with the tracked probe, and correct image reconstruction is checked on the workstation. Any mismatch warrants a new registration procedure or new intraoperative image acquisition and automated tracking/registration. (v) The positions of all tracked instruments and tools such as probes, drills, taps, screwdrivers are observed in real-time on the workstation.

Against this background, there is a need for efficiently and affordably controlling a surgical intervention to a bone allowing a comparably precise application of an instrument or tool to the bone at a predefined position and in a predefined orientation.

### Disclosure of the Invention

According to the invention this need is settled by a method as it is defined by the features of independent claim 1, by a system as it is defined by the features of independent claim 10 and by method as it is defined by the features of independent claim 14. Preferred embodiments are subject of the dependent claims.

In particular, the invention deals with a method of controlling a surgical intervention to a bone comprising: obtaining a three-dimensional image or multiplanar reconstruction of the bone, defining a position and an axis of intervention on the three-dimensional image or multiplanar reconstruction of the bone and controlling the orientation of an intervention instrument equipped with an orientation sensor during the surgical intervention by evaluating a signal provided by the orientation sensor. The method according to the invention further comprises referencing the intervention instrument with respect to the bone before the surgical intervention. This referencing is achieved by arranging the intervention instrument along and preferably also in contact with an anatomic landmark being an edge of the bone and rotating the orientation sensor into a predefined position, or by arranging the intervention instrument essentially perpendicular to an anatomic landmark being an essentially flat surface of the bone and rotating the orientation sensor into a predefined position.

Applying the surgical intervention to the bone can particularly relate to implanting a screw such as a pedicle screw or the like into the bone which can, e.g., be a spine or a single vertebra thereof or the like. The three-dimensional image or multiplanar reconstruction of the bone can be obtained by any suitable means such as by X-ray computed tomography (CT) magnetic resonance imaging (MRI), fluoroscopy or the like. It can be a digital image obtained in a computer running a computer program for implementing the method or parts thereof. The three-dimensional image or multiplanar reconstruction can also be comprised of three dimensional data without any graphical display. Furthermore, the three-dimensional image or multiplanar reconstruction of the bone can cover the complete bone or only relevant sections thereof. By defining the position and axis of intervention on the three-dimensional image or multiplanar reconstruction a preferred or ideal trajectory of intervention can be defined. For example, in spine surgery the trajectory of a pedicle screw to be applied into a vertebra can be defined taking into account the shape of the vertebra and the use of the screw. Such defining of the position and axis of intervention can particularly be performed on a computer wherein the computer can run a computer program providing appropriate tools for such definitions.

The term "signal" in connection with the orientation sensor can particularly relate to any data signal suitable for providing or transferring information about orientation in particular of orientation of the intervention instrument. Such information can, e.g. comprise X-, Y- and Z- coordinates, a tilt angle, a rotational angle, a sagittal angle being an angle in a sagittal plane of a body of a patient, an axial angle being an angle in a transversal plane of the body of the patient, a latero-medial angle, or any combinations thereof.

The predefined position into which the orientation sensor is rotated when referencing the intervention instrument can be a position aligned to a surgeon or operator wherein the surgeon or operator can be positioned quasi-parallel to the body of the involved patient such that the orientation sensor is approximately perpendicularly aligned with respect to the surgeon or operator. By referencing the intervention instrument or tool the orientation sensor allows for adjusting the intervention instrument with respect to the bone and with respect to the environment, e.g. in the world coordinate-system.

Controlling the orientation of the intervention instrument can particularly relate to monitoring the angulation of the intervention instrument, e.g. a sagittal angle and an axial angle thereof, and to displaying the real-time angulation to the surgeon compared to the pre-planned angulation. In particular, referencing the intervention instrument before the intervention by including a three-dimensional landmark situation, i.e. the edge of the bone or an orientation perpendicular to a flat bone portion, the intervention instrument can efficiently be spatially referenced. In many applications this allows for a sufficient allocation of the orientation of the intervention instrument with respect to the bone. Thus, in some embodiments it is sufficient to only control the angulation of the intervention instrument whereas the entry point or intervention position on the bone can comparably easily be found by the surgeon himself. Particularly within such embodiments, the method according to the invention allows for a comparably low-cost accurate control and application of the intervention.

Particularly in spinal surgery, the method according to the invention can additionally comprise confirming the situation at the bone such as a cephalo-caudal level with lateral fluoroscopy. Further, the method or substantial portions thereof can be performed on a computer such as a workstation, notebook, tablet, phablet or smartphone on which the orientation of the intervention instrument can be reproduced and embedded within a visualization of the surgical site. On the computer a computer program or software application can be executed on which the trajectories can easily be planned. This navigation of angles allows guiding the surgeon during the conventional technique of screw placement.. Furthermore, the method according to the invention can guide the surgeon to maintain a surgeon-determined orientation of the intervention instrument during a surgical maneuver comprising the intervention instrument.

The method according to the invention allows for efficiently achieving correct orientation and position of the intervention instrument in a bone surgical intervention. In particular, by ensuring that a correct starting point and a correct tilt, e.g. in a sagittal and axial plane, are applied which allows for warranting a correct trajectory of intervention, e.g. to implant a screw or the like into the bone without harming structures such as neurovascular structures around the bone. Furthermore, the method according to the invention provides an efficient real-time control at comparably low efforts. Particularly, since essential components involved are built around readily available technology of consumer electronics such as in smartphones, notebooks and computers its low cost can make it available to the global community of spine surgeons. Still further, with the method according to the invention radiation exposure to the operational staff as well as patients can be reduced, because thanks to the navigational support intermittent fluoroscopic checks may be omitted or at least reduced.

Preferably, evaluating the signal provided by the orientation sensor for controlling the orientation of the intervention instrument comprises comparing information obtained in the signal provided by the orientation sensor with information obtained when referencing the intervention instrument before the surgical intervention and with information obtained by defining the position and the axis of intervention on the three-dimensional image or multiplanar reconstruction of the bone. The information obtained by defining the position and the axis of intervention on the three-dimensional image or multiplanar reconstruction of the bone, i.e. the target information, can particularly comprise a target angulation such as a target sagittal angle and a target axial angle. The information obtained when referencing the intervention instrument before the surgical intervention, i.e. the reference information, can particularly comprise a reference angulation such as a reference sagittal angle and a reference axial angle. The information obtained in the signal provided by the orientation sensor, i.e. the real-time information, can particularly comprise a real-time angulation such as a real-time sagittal angle and a real-time axial angle. By comparing the real-time information with the reference information the real-time information can efficiently be transferred to be evaluable with regard to the target information. By comparing the transferred or referenced real-time information with the target information deviations between the orientation or position of the intervention instrument in relation to the pre-planned intervention orientation or defined axis of intervention can efficiently be detected.

Thereby, the method preferably comprises controlling the orientation of at least one further intervention instrument fixedly equipped with a further orientation sensor during the surgical intervention by evaluating a signal provided by the further orientation sensor wherein evaluating the signal provided by the further orientation sensor comprises comparing information obtained in the signal provided by the further orientation sensor with the information obtained when referencing the intervention instrument before the surgical intervention and with the information obtained by defining the position and the axis of intervention on the three-dimensional image or multiplanar reconstruction of the bone. Like this, the information obtained by referencing the intervention instrument can be transferred to the at least one further intervention instrument such that the intervention instrument and any further intervention instruments can be synchronized. It is therefore not required to reference the further intervention instruments or tools individually but the information obtained when initially referencing the intervention instrument can be also used for the further intervention instruments. Thus, efficiency of the method can be increased.

Preferably, controlling the orientation of the intervention instrument during the surgical intervention comprises displaying information about a deviation between the orientation of the intervention instrument and the defined axis of intervention. Such displaying can be based on preoperative three-dimensional image of the bone, a multiplanar reconstruction thereofor a surgeon-controlled initial trajectory established by respecting intraoperative anatomical landmarks and if necessary fluoroscopic control. In this manner, the surgeon can be informed about the orientation of intervention in real-time which allows him to continuously take appropriate corrections, without the need for checking the orientation of intervention with repetitive fluoroscopies.

Preferably, the orientation sensor comprises an accelerometer and the signal provided by the orientation sensor comprises accelerometer information. Such an accelerometer or three axis accelerometer such as the compact low-power three axes linear accelerometer available as STMicroelectronics LIS302DL allows for comparably precise angular measurement and particularly absolute angular measurement such that conclusions about the orientation of the intervention instrument can be drawn. Furthermore, accelerometers are available at comparably low costs and in comparably small dimensions.

Preferably, the orientation sensor comprises a gyroscope and the signal provided by the orientation sensor comprises gyroscope information. Such a gyroscope or three axis gyro like the gyro available as STMicroelectronics L3G4200D allows for measuring angular velocity in a comparably fast manner. Like this, whereas absolute angle measurements usually are not possible with gyros angular changes can be efficiently detected, e.g., by means of integration. Furthermore, gyroscopes are available at comparably low costs and in comparably small dimensions.

Preferably, the orientation sensor comprises a magnetometer and the signal provided by the orientation sensor comprises magnetometer information. Such magnetometer allows for comparably precisely measuring a three-dimensional orientation of the sensor such that conclusions about the orientation of the intervention instrument can be drawn.

In a preferred embodiment the orientation sensor comprises a combination of accelerometer, gyroscope and/or magnetometer and particularly all three of it. In such arrangements the best of all three kinds of sensing can be combined. For example, accelerometers typically are comparably precise but slow whereas gyroscopes typically are comparably fast. The combination allows for a cost-effective, compact angle measurement.

Preferably, the position into which the orientation sensor is rotated is predefined in relation to a surgeon or patient. Since the surgeon or operator usually is handling the intervention instrument, this allows an efficient and handy referencing of the intervention instrument with respect to the bone when the anatomic landmark is the essentially flat surface of the bone.

Preferably, controlling the orientation of the intervention instrument comprises evaluating a bone reference signal provided by a bone orientation sensor being releasably attached to the bone. Such control can be particularly beneficial in situations where the bone is difficult to situate with respect to an operating room when lying in a prone position. By attaching the bone orientation sensor to the bone, the angulation of the intervention instrument can be controlled relative to the bone. Thereby, any movement of the bone can be compensated.

Preferably, controlling the orientation of the intervention instrument is determined by an optical means.

A further aspect of the invention relates to a surgical intervention system for applying a surgical intervention to a bone. The surgical intervention system comprises a computer program with computer readable commands causing a computer to implement an embodiment of the method described above when being loaded to or executed by the computer. Such a computer program allows for efficiently implementing the method according to the invention. Thus, the effects and benefits described above and below in connection with the methods according to the invention and its preferred embodiments can be realized in an efficient and effective manner.

Preferably, the surgical intervention system comprises a computer executing the computer program and an orientation sensor mountable to an intervention instrument at a predefined position, the orientation sensor being connected to the computer for transferring a signal from the orientation sensor to the computer, wherein the orientation sensor is arranged for transferring the signal comprising orientation information to the computer and the computer is arranged for evaluating the signal received from the orientation sensor for controlling the orientation of the intervention instrument. Thereby, for connecting the orientation sensor with the computer the orientation sensor preferably comprises a wireless sender and the computer a wireless receiver.

Preferably, the orientation sensor of the surgical intervention system comprises an accelerometer, a gyroscope, a magnetometer or any combination thereof or an optical orientation sensor and the signal provided by the orientation sensor comprises respective information.

Another further aspect of the invention relates to a method of a surgical intervention to a bone using a surgical intervention system as described above. This method of surgical intervention comprises: by means of a computer defining a position and an axis of intervention on a three-dimensional image or multiplanar reconstruction of the bone, by means of the computer identifying an anatomic landmark of the bone on the three-dimensional image or multiplanar reconstruction of the bone, referencing an intervention instrument fixedly equipped with an orientation sensor with respect to the bone; by arranging the intervention instrument along and preferably also in contact with the anatomic landmark being an edge of the bone and rotating the orientation sensor into a predefined position, or by arranging the intervention instrument essentially perpendicular to an anatomic landmark being an essentially flat surface of the bone and rotating the orientation sensor into a predefined position; by means of the intervention instrument applying a surgical intervention to the bone; and by means of the computer controlling the orientation of the intervention instrument during the surgical intervention wherein the computer evaluates a signal provided by the orientation sensor. Such a method allows for an efficient and precise surgical intervention to the bone with comparably cost effective equipment.

These and other aspects of the invention will be apparent from and elucidated with reference to the embodiment(s) described hereinafter.

### Brief Description of the Drawings

The invention is described in more detail hereinbelow by way of exemplary embodiments and with reference to the attached drawings, in which:
- Fig. 1: shows a perspective view of a pedicle finder as an intervention instrument being referenced in an embodiment of the method of controlling a surgical intervention to a bone according to the invention and an embodiment of the method of a surgical intervention according to the invention;
- Fig. 2: shows a schematic view of an embodiment of surgical intervention system according to the invention as used in the method of controlling surgical intervention to a bone of Fig. 1 and the method of surgical intervention of Fig. 1;
- Fig. 3: shows a flow scheme of the method of controlling surgical intervention to a bone of Fig. 1 and the method of surgical intervention of Fig. 1;
- Fig. 4: shows a perspective view of the pedicle finder of Fig. 1 when being at an entry point of a vertebra; and
- Fig. 5: shows a perspective view of a screwdriver as an intervention instrument applying a pedicle screw to the vertebra in the method of controlling surgical intervention to a bone of Fig. 1 and the method of surgical intervention of Fig. 1.

### Description of Embodiments

The following applies to the following description. If, in order to clarify the drawings, a figure contains reference signs which are not explained in the directly associated part of the description, then it is referred to previous description sections.

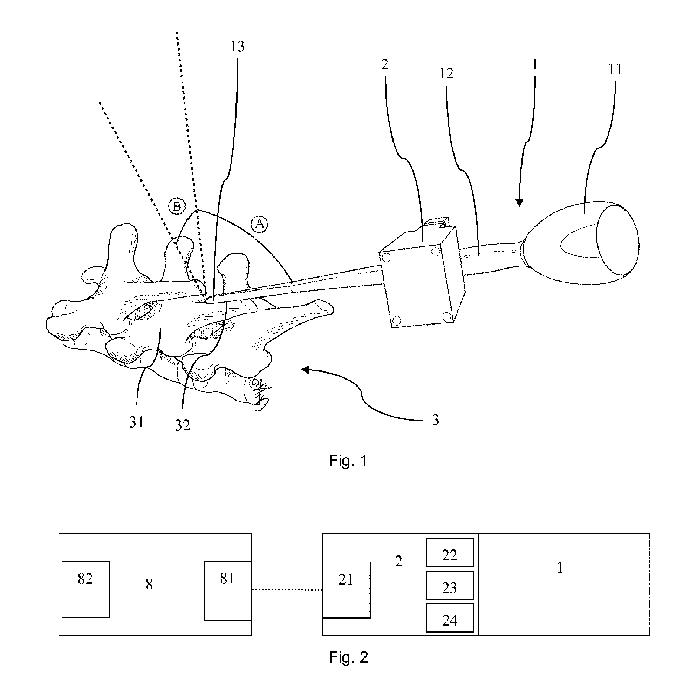

Fig. 1 shows a pedicle finder 1 as an intervention instrument when being referenced in an embodiment of the method of controlling a surgical intervention to a spine 3 as a bone according to the invention and an embodiment of the method of a surgical intervention according to the invention applied with a surgical intervention system according to the invention. The pedicle finder 1 comprises a grip 11, a shaft 12 and tapered tip 13. The grip 11 is mounted to one longitudinal end of the shaft 12 which has the tapered tip 13 on the opposite side. The pedicle finder 1 is equipped with an orientation sensor 2 having an accelerometer, a gyroscope and a magnetometer. The spine comprises vertebrae 31 most having spinous processes or cranial edges 32.

In Fig. 2 a computer 8 of the surgical intervention system is shown wherein the surgical intervention is controlled by means of the computer 8. The computer 8 has a processing unit (CPU), a memory and a data storage. It is executing a computer program 82 for arranging the computer 8 to control the surgical intervention. Further, the computer comprises a wireless receiver 81 which can be connected to a wireless sender 21 of the orientation sensor 2 mounted to the pedicle finder 1. The orientation sensor 2 has an accelerometer 22, a gyroscope 23 and a magnetometer 24. It is arranged to provide a signal via its wireless sender 81 and the wireless receiver 81 to the computer 8. Thereby, the signal comprises accelerometer information gathered by the accelerometer 22, gyroscope information gathered by the gyroscope 23 and magnetometer information gathered by the magnetometer 24.

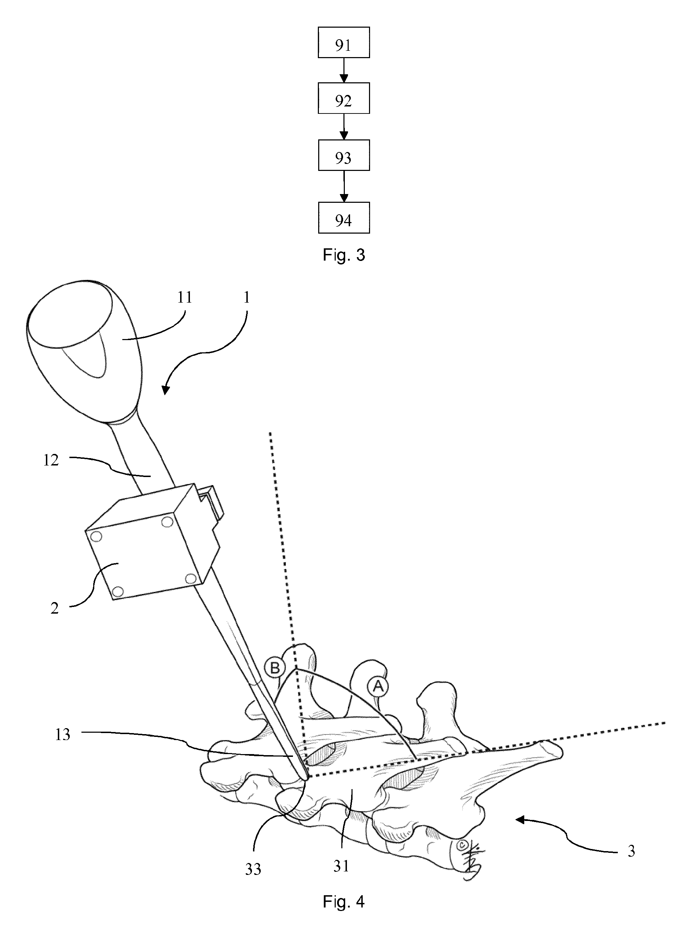

As shown in Fig. 3, controlling of the surgical intervention comprises four steps 91, 92 93 and 94. In the first step 91 a three dimensional image or multiplanar reconstruction of the spine 3 is obtained. This can, e.g., be performed by means of computed tomography (CT) or a similar technology. The obtained image is loaded to the computer 8 and displayed on a screen to a surgeon or operator. In the second step 92 the surgeon defines an entry point 33 (see Fig. 4) as position and an axis of intervention on the three dimensional image displayed by the computer 8. This position and axis of intervention, i.e. the target information, can particularly comprise a target angulation such as a target sagittal angle and a target axial angle. For this, the computer program 81 provides appropriate tools allowing the surgeon or operator to fulfil the required tasks. Alternatively, the surgeon may choose to manually measure the target information (target sagittal angle and a target axial angle) on a three dimensional image or multiplanar reconstruction of the spine 3.

In the third step 93, the pedicle finder 1 is referenced 93 or zeroed. As can be best seen in Fig. 1, for that purpose the tapered tip 13 and proximal shaft 12 of the pedicle finder 1 is arranged along one of the cranial edges 32 of the target vertebra 31 of the vertebrae 31 of the spine 3, i.e. the corresponding dorsal edges of the spinous process 32 of the target vertebra. In this position, the orientation sensor 2 is rotated around a longitudinal axis of the pedicle finder 1 until it is oriented into the direction of the surgeon as predefined position. Thereby, the surgeon or operator can be positioned quasi-parallel to the body of the involved patient such that the orientation sensor 2 is approximately perpendicularly aligned with respect to the surgeon or operator. In this situation, reference data comprising a reference sagittal angle A and a reference axial angle B or transversal angle of a reference position and orientation or the zero position is evaluated and stored in the computer 8 and/or in the orientation sensor 2.

As shown in Fig. 4, in the fourth step 94 the tapered tip 13 of the pedicle finder 1 is positioned and applied at the entry point 33 of the vertebra 3. Thereby, the orientation of the pedicle finder 1 is controlled during this application or surgical intervention. The orientation sensor 2 continuously provides the signal comprising the accelerometer, gyroscope and magnetometer information to the computer 8. The computer 8 evaluates the signal with regard to the real-time sagittal angle A and axial angle B and provides the surgeon with information about the orientation of the pedicle finder 1. In particular, the computer calculates and detects deviations between the real-time orientation and position of the pedicle finder 1 and the predefined or target orientation and position and warns or informs the surgeon respectively.

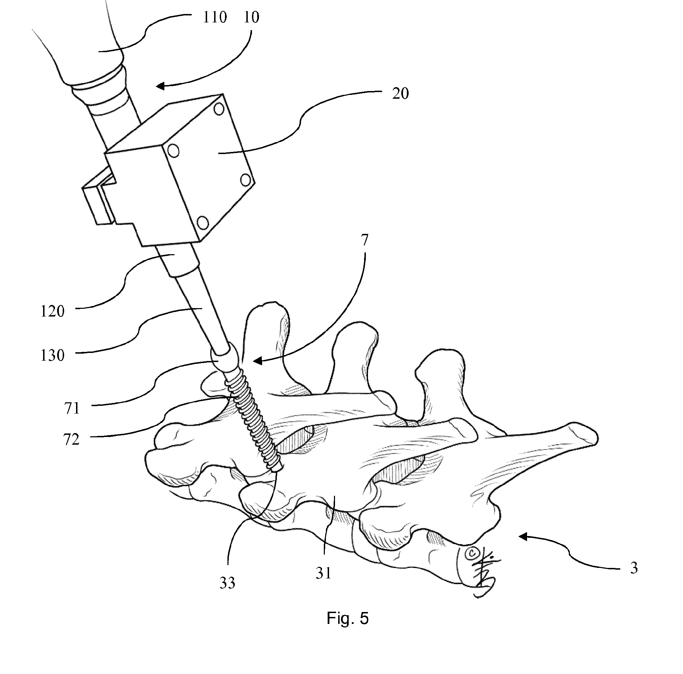

Fig. 5 shows a screwdriver 10 as another embodiment of an intervention instrument used in the surgical intervention to the spine 3. The screwdriver 10 comprises a grip 110, a shaft 120 and male head 130. The grip 110 is mounted to one longitudinal end of the shaft 120 and the male head 130 to the other longitudinal end of the shaft 120. The screwdriver 10 is equipped with an orientation sensor 20 having an accelerometer, a gyroscope and a magnetometer. Application and control of the screwdriver 10 is identically performed as described with respect to the pedicle finder 1 above. In particular, the screwdriver is in the step 93 referenced and in the step 94 positioned and applied at the entry point 33 of the vertebra 3. Steps 91 and 92 do not have to be repeated when the intervention instrument is changed from the pedicle finder 1 to the screwdriver 10. A pedicle screw 7 is placed at the entry point 33 prepared by the pedicle finder 1. The male head 130 of the screwdriver 10 engages in a corresponding female head 71 of the screw 7. By turning the screwdriver 10 around its longitudinal axis a threaded shaft 72 of the screw 7 is forwarded into the vertebra 31. Thereby, in the step 94 orientation of the screwdriver 10 and, thus, the trajectory of the screw is ongoingly controlled as described above in connection with control of the orientation of the pedicle finder 1.

While the invention has been illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below. For example, further applications of the invention can comprise:
- Connecting firmly two intervention instruments, each comprising a sensor, to the proximal and distal ends of, for instance, an instrumented but precorrected deformity (i.e. kyphosis before rod insertion and correction of deformity), the angular correction during deformity-reducing maneuvers or closing of a bone wedge osteotomy can be monitored in real time, whereby the relative angular change of each sensor at the distal and proximal anchored intervention instrument is received by the computer for calculating the total angle of correction.
- Alining the intervention instrument with sensor sequentially with the cranial and caudal plane of a developing osteotomy (i.e. bone wedge removal before posterior subtraction osteotomy) the magnitude of the wedge osteotomy is displayed in real-time.

The invention also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims ort the exemplary embodiments as well as subject matter comprising said features.

Furthermore, in the claims the word "comprising" does not exclude other elements or steps, and the indefinite article "a" or "an" does not exclude a plurality. A single unit or step may fulfill the functions of several features recited in the claims. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Any reference signs in the claims should not be construed as limiting the scope.

A computer program may be stored/distributed on a suitable medium, such as an optical storage medium or a solid-state medium supplied together with or as part of other hardware, but may also be distributed in other forms, such as via the Internet or other wired or wireless telecommunication systems. In particular, e.g., a computer program can be a computer program product stored on a computer readable medium which computer program product can have computer executable program code adapted to be executed to implement a specific method such as the method according to the invention. Furthermore, a computer program can also be a data structure product or a signal for embodying a specific method such as a method according to the invention.

## Claims

1. Method of controlling a surgical intervention to a bone (3), comprising
obtaining a three-dimensional image or multiplanar reconstruction of the bone (3),
defining a position and an axis of intervention on the three-dimensional image of the bone or multiplanar reconstruction (3), and
controlling the orientation of an intervention instrument (1; 10) equipped with an orientation sensor (2; 20) during the surgical intervention by evaluating a signal provided by the orientation sensor (2; 20),
**characterized in** further comprising
referencing the intervention instrument (1; 10) with respect to the bone (3) before the surgical intervention
by arranging the intervention instrument (1; 10) along an anatomic landmark (32) being an edge of the bone (3) and rotating the orientation sensor (2; 20) into a predefined position, or
by arranging the intervention instrument (1; 10) essentially perpendicular to an anatomic landmark (32) being an essentially flat surface of the bone (3) and rotating the orientation sensor (2; 20) into a predefined position.

2. Method according to claim 1, wherein evaluating the signal provided by the orientation sensor (2; 20) for controlling the orientation of the intervention instrument (1; 10) comprises comparing information obtained in the signal provided by the orientation sensor (2; 20) with information obtained when referencing the intervention instrument (1; 10) before the surgical intervention and with information obtained by defining the position and the axis of intervention on the three-dimensional image or multiplanar reconstruction of the bone (3).

3. Method according to claim 2, comprising controlling the orientation of at least one further intervention instrument (1; 10) fixedly equipped with a further orientation sensor (2; 20) during the surgical intervention by evaluating a signal provided by the further orientation sensor (2; 20) wherein evaluating the signal provided by the further orientation sensor (2; 20) comprises comparing information obtained in the signal provided by the further orientation sensor (2; 20) with the information obtained when referencing the intervention instrument (1; 10) before the surgical intervention and with the information obtained by defining the position and the axis of intervention on the three-dimensional image or multiplanar reconstruction of the bone (3).

4. Method according to any one of the preceding claims, wherein controlling the orientation of the intervention instrument (1; 10) during the surgical intervention comprises displaying information about a deviation between the orientation of the intervention instrument (1; 10) and the defined axis of intervention.

5. Method according to any one of the preceding claims, wherein the orientation sensor (2; 20) comprises an accelerometer (22) and the signal provided by the orientation sensor (2; 20) comprises accelerometer information.

6. Method according to any one of the preceding claims, wherein the orientation sensor (2; 20) comprises a gyroscope (23) and the signal provided by the orientation sensor (2; 20) comprises gyroscope information.

7. Method according to any one of the preceding claims, wherein the orientation sensor (2; 20) comprises a magnetometer (24) and the signal provided by the orientation sensor (2; 20) comprises magnetometer information.

8. Method according to any one of the preceding claims, wherein the position into which the orientation sensor (2; 20) is rotated is predefined in relation to a surgeon or patient.

9. Method according to any one of the preceding claims, wherein controlling the orientation of the intervention instrument (1; 10) comprises evaluating a bone reference signal provided by a bone orientation sensor (2; 20) being releasably attached to the bone (3).

10. Method according to any one of the preceding claims, wherein controlling the orientation of the intervention instrument (1; 10) is determined by an optical means.

11. Surgical intervention system for applying a surgical intervention to a bone (3) comprising a computer program (82) with computer readable commands causing a computer (8) to implement a method of any one of the preceding claims when being loaded to or executed by the computer (8).

12. Surgical intervention system according to claim 11, comprising a computer (8) executing the computer program and an orientation sensor (2; 20) mountable to an intervention instrument (1; 10) at a predefined position the orientation sensor (2; 20) being connected to the computer (8) for transferring a signal from the orientation sensor (2; 20) to the computer, wherein the orientation sensor (2; 20) is arranged for transferring the signal comprising orientation information to the computer (8) and the computer (8) is arranged for evaluating the signal received from the orientation sensor (2; 20) for controlling the orientation of the intervention instrument (1; 10).

13. Surgical intervention system according to claim 12, wherein for connecting the orientation sensor (2; 20) with the computer the orientation sensor (2; 20) comprises a wireless sender (21) and the computer (8) comprises a wireless receiver (81).

14. Surgical intervention system according to any one of claims 11 to 13, wherein the orientation sensor (2; 20) comprises an accelerometer (22), a gyroscope (23), a magnetometer (24) or any combination thereof or an optical orientation sensor and the signal provided by the orientation sensor (2; 20) comprises respective information.

15. Method of a surgical intervention to a bone (3) using a surgical intervention system according to any one of claims 11 to 14, comprising
by means of a computer (8) defining a position and an axis of intervention on a three-dimensional image or multiplanar reconstruction of the bone (3);
by means of the computer (8) identifying an anatomic landmark (32) of the bone (3) on the three-dimensional image or multiplanar reconstruction of the bone (3);
referencing an intervention instrument (1; 10) fixedly equipped with an orientation sensor (2; 20) with respect to the bone (3);
by arranging the intervention instrument (1; 10) along the anatomic landmark (32) being an edge of the bone (3) and rotating the orientation sensor (2; 20) into a predefined position, or
by arranging the intervention instrument (1; 10) essentially perpendicular to an anatomic landmark (32) being an essentially flat surface of the bone (3) and rotating the orientation sensor (2; 20) into a predefined position;
by means of the intervention instrument (1; 10) applying a surgical intervention to the bone (3); and
by means of the computer (8) controlling the orientation of the intervention instrument (1; 10) during the surgical intervention wherein the computer (8) evaluates a signal provided by the orientation sensor (2; 20).
